(19) Europäisches Patentamt
European Patent Office
Office européen des brevets

(11) EP 0 902 840 B1

(12) **FASCICULE DE BREVET EUROPEEN**

(45) Date de publication et mention de la délivrance du brevet:
**05.06.2002 Bulletin 2002/23**

(21) Numéro de dépôt: **97907151.1**

(22) Date de dépôt: **28.02.1997**

(51) Int Cl.[7]: **C12Q 1/68**, C07H 21/04, C12P 19/34, G01N 33/76, G01N 33/574

(86) Numéro de dépôt international:
**PCT/FR97/00361**

(87) Numéro de publication internationale:
**WO 97/32997 (12.09.1997 Gazette 1997/39)**

(54) **PROCEDE POUR LA DETECTION DE LA TRANSFORMATION MALIGNE DE CELLULES HUMAINES**

VERFAHREN ZUR ERKENNUNG VON MALIGNEN TRANSFORMIERTEN MENSCHLICHEN ZELLEN

MALIGNANT HUMAN CELL TRANSFORMATION DETECTION METHOD

(84) Etats contractants désignés:
**AT BE CH DE DK ES FI FR GB GR IE IT LI LU MC NL PT SE**

(30) Priorité: **04.03.1996 FR 9602683**

(43) Date de publication de la demande:
**24.03.1999 Bulletin 1999/12**

(73) Titulaire: **Institut Gustave Roussy**
**F-94800 Villejuif (FR)**

(72) Inventeurs:
- **BELLET, Dominique**
  **F-75014 Paris (FR)**
- **BIDART, Jean-Michel**
  **F-92330 Sceaux (FR)**
- **VIDAUD, Michel**
  **F-75014 Paris (FR)**
- **LAZAR, Vladimir**
  **94800 Villejuif (FR)**

(74) Mandataire: **Warcoin, Jacques et al**
**Cabinet Régimbeau**
**20, rue de Chazelles**
**75847 Paris cedex 17 (FR)**

(56) Documents cités:
**WO-A-94/20859**

- **CANCER RESEARCH, vol. 57, Janvier 1997, pages 516-23, XP002040116 BELLET D ET AL: "Malignant transformation of nontrophoblastic cells is associated with the expression of Chorionic gonadotropin beta genes normally transcribed in trophoblastic cells"**
- **CANCER RESEARCH, vol. 55, no. 17, 1 Septembre 1995, pages 3735-8, XP000608909 LAZAR V., ET AL.: "EXPRESSION OF HUMAN CHORIONIC GONADOTROPHIN BETA SUBUNIT GENES IN SUPERFICIAL AND INVASIVE BLADDER CARCINOMAS" cité dans la demande**
- **THE BIOCHEMICAL JOURNAL, vol. 263, Octobre 1989, pages 1-10, XP000400720 COTTON G.H.: "Detection of single base changes in nucleic acids"**
- **INTERNATIONAL JOURNAL OF CANCER, vol. 63, 27 Novembre 1995, pages 633-38, XP000609237 HOUSSEAU F., ET AL.: "Reaction of peripheral blood lymphocytes to the human chorionic gonadotrophin beta sub-unit in patients with productive tumors"**
- **TRENDS IN ENDOCRINOL. METABOLISM, vol. 4, no. 9, Novembre 1993, pages 285-91, XP000609256 BIDART J., ET AL: "human chorionic gonadotrophin: Molecular forms, detection, and clinical implications"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 902 840 B1

Printed by Jouve, 75001 PARIS (FR)

## Description

La présente invention concerne un procédé pour la détection de la transformation maligne des cellules et son application pour le diagnostic, la surveillance et l'établissement du pronostic des cancers.

L'hormone chorionique gonadotrope (hCG) est une glycoprotéine formée de deux sous-unités dénommées alpha (hCGα) et beta (hCGβ) et liées de façon non covalente (1). Au cours de la grossesse, les cellules trophoblastiques du placenta produisent l'hCG dimérique et les sous-unités hCGα ou hCGβ libres qui sont retrouvées en quantité importante dans le sérum. Le développement de techniques de dosage sensibles et spécifiques de l'hCG et, indépendamment, de la sous-unité hCGα libre et de la sous-unité hCGβ libre a permis de montrer que des taux sériques d'hCG jusqu'à 1 000 pg/ml et des taux sériques de sous-unité hCGα libre jusqu'à 3 000 pg/ml étaient présents en dehors de la grossesse chez des sujets sains (2,3). De plus, il a été observé que des taux sériques d'hCG ou de sous-unité hCGα libre supérieurs à ces valeurs usuelles sont principalement trouvés chez les malades avec une tumeur testiculaire ou ovarienne d'origine trophoblastique.

En contraste, de faibles taux sériques de sous-unité hCGβ libre, inférieurs à 100 pg/ml, sont détectables chez les sujets sains en dehors de la grossesse. Des taux sériques d'hCGβ libre supérieurs à ces valeurs usuelles sont retrouvés chez de nombreux patients cancéreux porteurs d'une tumeur d'origine gonadique ou non gonadique (2). En particulier, 47% des patients avec un cancer de la vessie, 32% des patients avec un cancer du pancréas ct 30% des patients avec un cancer du col de l'utérus ont un taux sérique d'hCGβ libre supérieur à 100 pg/ml (2). Chez les patients avec un cancer de la vessie, la présence de taux élevé (supérieur à 100 pg/ml) d'hCGβ libre serait corrélée avec une évolution défavorable de la maladie (4, 5).

Si la sous-unité hCGα est codée par un seul gène situé sur le chromosome 6q21.1-q23, la sous-unité hCGβ a été décrite comme codée par une famille de gènes localisés sur le chromosome 19q13.3. A la suite de nombreuses études il a été montré qu'il existait 7 gènes CGβ dénommés CGβ7 ou β7, CGβ6 ou β6, CGβ8 ou β8, CGβ5 ou β5, CGβ1 ou β1, CGβ2 ou β2, CGβ3 ou β3 (6). Les gènes β6 et β7 sont alléliques (7).

Seuls les gènes β7, β6, β8, β5 et β3 sont susceptibles de coder pour la sous-unité hCGβ composée de 145 acides aminés. Les gènes β1 et β2 se caractérisent par la présence :

- d'une insertion d'environ 770 nucléotides dans la partie 5' des gènes CGβ,
- d'une mutation ponctuelle du site 5' d'épissage du premier intron des gènes CGβ.

Il a été montré que les gènes β1 et β2 sont susceptibles d'être transcrits dans un certain nombre de tissus et auraient la capacité de coder une protéine de 132 acides aminés de séquence différente par rapport à celle de l'hCGβ (7).

Le séquençage de tout ou partie des gènes β7, β6, β5 et β3 et les cartographies de restriction ont montré que les nucléotides correspondant aux codons 2, 4 et 117 par rapport à la protéine CGβ mature étaient différents selon les gènes en cause (8, 9, 10).

L'analyse des séquences nucléotidiques pour les positions équivalentes a été réalisée pour le gène β2 et il a été montré qu'il présentait les mêmes caractéristiques que le gène β6. En revanche, pour le gène β1, seule la position 117 a été déduite de l'analyse de cartographie de restriction et a été considérée comme étant "de type Asp".

Par ailleurs, l'expression des différents gènes CGβ a été analysée de façon semi-quantitative dans le tissu placentaire. Les résultats trouvés montrent que les transcrits β5 sont en quantité très supérieure par rapport aux transcrits β3 et β8 Les transcrits β7, β1 et β2 sont très minoritaires (7). Enfin, plusieurs études ont été réalisées pour rechercher les transcrits CGβ dans différents tissus normaux ou tumoraux non trophoblastiques ou sur différentes lignées cellulaires dérivées de tissus cancéreux d'origine trophoblastique et non trophoblastique (11-17). Les techniques utilisées au cours de ces différentes études n'ont pas permis de distinguer les transcrits β7, β8, β5 et β3. Ces études ont montré que des transcrits β7, β8, β5 ou β3 sont notamment présents dans le testicule normal (11), le testicule tumoral (12), la vessie tumorale (13), le placenta normal, des lignées de choriocarcinome placentaire (14-16) et dans diverses lignées dérivées de cellules tumorales non trophoblastiques (15-17).

Récemment, une étude a été réalisée sur des tissus normaux et tumoraux d'origine vésicale pour quantifier les transcrits β7, β8, β5 et β3. Cette étude était basée sur le profil observé après amplification des exons 1 et 2 et coupure enzymatique dans des positions nucléotidiques correspondant à la partie 5' transcrite non traduite de l'exon 1 (18). Cette étude a montré que le tissu vésical normal exprime exclusivement le gène β7 et que la transformation maligne du tissu vésical s'accompagne de l'acquisition de l'expression des gènes β8 et/ou β5 et/ou β3 dans 45 à 95% des cas (18).

La présente invention repose sur la mise en évidence de l'importance des gènes β3, β5, β8 et β9 dans le passage au processus malin, notamment par rapport à l'expression des produits des gènes β6 et β7 qui existe dans les tissus normaux.

La présente invention concerne un procédé pour la détection de la transformation maligne de cellules humaines, caractérisé en ce qu'on met en évidence dans lesdites cellules une surexpression des produits des gènes β3, β5, β8 et/ou β9 codant pour la sous-unité hCGβ par rapport à leur expression dans les cellules non malignes.

Il est important de noter que le gène β9 qui est allélique du gène β3 est décrit ci-après pour la première fois et que, comme les gènes β3, β5 et β8, il conduit à la synthèse d'une sous-unité hCGβ dont la position 117 est un acide aspartique et est impliquée dans le processus malin.

Par "produits d'expression des gènes β3, β5, β8 et/ou β9", on entend désigner aussi bien les produits naturels de transcription ARNm que les produits naturels de traduction, soit l'hCGβ.

S'il est intéressant de mettre en évidence les produits de ces gènes β3, β5, β8 et β9 et leur surexpression par rapport à des tissus normaux ou à des normes prédéterminées, il peut être encore plus intéressant de mettre en évidence la variation d'un indice qui implique le rapport entre les produits d'expression des gènes β3, β5, β8 et β9 et l'ensemble des mêmes produits d'expression pour tous les gènes β dans les mêmes tissus.

Cet indice dit "indice de transformation" sera détaillé dans ce qui va suivre.

Dans un premier mode de mise en oeuvre du procédé selon l'invention, on met en évidence les transcrits des gènes β, c'est-à-dire les ARNm.

Comme cela est possible, on peut envisager de mesurer séparément les transcrits des gènes β3, β5, β8 et β9 ou de les doser par groupe en fonction de leur structure nucléotidique. Néanmoins, on peut, avantageusement, mettre à profit chez ces gènes l'existence d'une séquence GAC codant pour l'acide aspartique en position 117, qui se situe de 774 à 776 et dont la position 775 diffère de la position correspondante des gènes β6 et β7 codant pour Ala117 par un seul nucléotide, A est remplacé par C. Les positions sont indiquées par rapport à l'ARNm mature, le site d'initiation de la transcription étant numéroté +1. Il existe dans la littérature des résultats contradictoires concernant la position exacte de l'initiation de la transcription des gènes CGβ. On a utilisé celle proposée par Otani et al. (19). Le produit d'expression des gènes β6 et β7 et présent dans les cellules normales.

On peut donc mettre en évidence la surtranscription des transcrits des gènes β3, β5, β8 ct β9 en mesurant uniquement la surtranscription des transcrits des gènes β qui comportent un A en position 775.

Dans ce cas, on met en évidence la présence des transcrits des gènes β3, β5, β8 et/ou β9 comportant la séquence codant pour l'acide aspartique en position 774 à 777.

Afin d'éliminer au maximum les problèmes liés aux méthodes de dosage, on utilisera de préférence l'indice de transformation qui mesure le rapport entre les transcrits comportant un A en position 775 et l'ensemble des transcrits des gènes β, soit :

$$\text{Indice CG117} = \frac{\text{transcrits GA}^{775}\text{C}}{\text{transcrits GA}^{775}\text{C + transcrits GC}^{775}\text{C}} \text{ X 100}$$

La détermination des transcrits nécessite, en général, une étape d'amplification, bien que celle-ci puisse ne pas être nécessaire.

Ce dosage nucléotidique peut être mis en oeuvre grâce à des méthodes d'amplification, soit sur ARN transcrit, soit plus commodément sur ADNc après transcription inverse des ARNm en utilisant, par exemple, la méthode PCR.

Plus particulièrement, dans ce procédé, pour mettre en évidence la surexpression des transcrits :

(a) on effectue d'abord une réverse transcription des ARNm totaux,
(b) on amplifie sélectivement les ADNc des gènes codant la sous-unité β de l'hCG, et plus particulièrement les fragments portant la séquence 774 à 776, et
(c) on met en évidence la présence de produits d'amplification comportant un A en position 775 que l'on compare avec le même dosage effectué sur des tissus normaux ou par rapport à des standards.

De préférence, on mettra en évidence dans l'étape (c) également la présence de produits d'amplification comportant un C en position 775 et on calculera l'indice de transformation. C'est la variation de cet indice par rapport à des tissus normaux ou à des normes qui fournira un diagnostic ou un pronostic sur le passage au stade malin.

La révélation, après amplification, peut être réalisée par une élongation des produits d'amplification à partir d'amorces marquées, l'une des amorces comportant à l'extrémité 3' et à la position correspondant au nucléotide 775 un C et l'autre comportant dans la méme position un A avec un marquage différent.

On obtient ainsi des produits d'extension correspondant à la présence d'un A en position 775 et respectivement à la présence d'un C en position 775. En effet, lorsqu'il n'y a pas d'appariement possible de l'amorce et du produit d'amplification à l'extrémité 3', l'extension est très difficile, comme cela sera expliqué plus en détail dans les exemples.

Il est ainsi possible de mesurer en une seule opération les hCG A775 et la somme des produits A775 et C775.

On peut également utiliser des méthodes différentes type LCR ou NASBA ou même des techniques d'hybridation directe, lesquelles peuvent être adaptées aux principes décrits précédemment.

La mise en oeuvre de ce procédé a montré que, à l'exception du placenta, l'indice est égal à 0% dans la plupart des tissus sains dans lesquels il est possible de détecter des transcrits CGβ par RT-PCR, mais que la transformation maligne des tissus peut s'accompagner d'une élévation de cet indice. En particulier, une élévation de cet indice a été observée dans des cancers de forte incidence tels que les cancers du colon, du sein, de la vessie, de la prostate et de la thyroïde par exemple.

Si la méthode précédente est la méthode de choix pour la mise en oeuvre du procédé, il est également possible de prévoir la mise en évidence des produits de traduction des gènes en cause.

Il est possible de mettre en évidence la surexpression des produits des gènes β3, β5, β8 et β9 en mettant en évidence la présence d'une hCGβ portant l'acide aspartique en position 117 à l'aide d'un anticorps monoclonal spécifique de l'épitope correspondant. En effet, les gènes β7 et β6 qui ne sont pas des marqueurs de la transformation tumorale codent pour une hCGβ qui présente une alanine en position 117 et qui possède donc un épitope différent. Ainsi, par "anticorps spécifique" on entend désigner un anticorps qui reconnaît l'épitope à l'Aspll7 et ne reconnaît pas l'épitope à Ala117.

La mise en évidence peut être effectuée par l'une des méthodes connues en immunologie telles que l'ELISA, RIA ou IRMA par exemple.

Comme précédemment, il peut être intéressant, afin d'éviter d'éventuels problèmes liés à des particularités des dosages, d'effectuer cette mesure sous forme d'un indice de transformation qui, comme précédemment, serait le rapport entre la mesure effectuée sur la position Asp117(hCGβ) et la mesure de (hCGβ) totale.

Le procédé selon l'invention présente, notamment sous forme de dosage nucléotidique, l'avantage d'être basé sur un changement génétique simple qui est très fortement corrélé avec le phénotype malin.

Ce test présente l'intérêt de pouvoir être réalisé sur un petit nombre de cellules exfoliées.

Ce test peut être aussi bien quantitatif que qualitatif, simple et sensible, il peut être réalisé en moins de 3 heures.

Il permet le diagnostic précoce de la plupart des cancers à forte prédominance sur des échantillons très divers :

- biopsie pour le sein et la prostate,
- selles pour le cancer du colon,
- sécrétion vaginale (ovaire),
- urine (vessie),
- expectorat (poumon),
- sang total (cellules mononucléées du sang circulant pour le diagnostic de cellules tumorales circulantes).

Le dosage qualitatif permet également d'évaluer le stade de la tumeur et son pronostic.

Afin de mieux comprendre les exemples ci-après, le schéma suivant schématise l'organisation des gènes hCGβ :

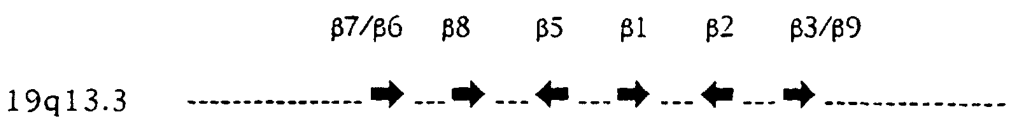


et le tableau 1 ci-après rassemblent les informations sur ces différents gènes.

La transcription et la traduction des gènes β1 et β2, si elles existent, ne conduisent pas à la synthèse d'une sous-unité hCGβ de 145 acides aminés.

Les gènes β3, β5, β8 et β9 conduisent à la synthèse d'une sous-unité hCGβ de 145 acides aminés ayant en position 117 un résidu acide aspartique.

Les gènes β6 et β7 conduisent à la synthèse d'une sous-unité hCGβ de 145 acides aminés ayant en position 117 un résidu alanine.

Tableau 1

| Codons | β7 | β6 | β8 | β5 | β1 | β2 | β3 | β9 |
|---|---|---|---|---|---|---|---|---|
| | Gènes | | | | | | | |
| 2 | AGG | AAG | AAG | AAG | AAG | AAG | AAG | AAG |
| | Arg | Lys | Lys | Lys | | | Lys | Lys |
| 4 | ATG | CCA | CCG | CCG | CCG | CCG | CCG | CCG |
| | Met | Pro | Pro | Pro | | | Pro | Pro |
| 117 | GCC | GCC | GAC | GAC | GCC | GAC | GAC | GAC |
| | Ala | Ala | Asp | Asp | | | Asp | Asp |

NB : Les données encadrées correspondent aux données nouvelles

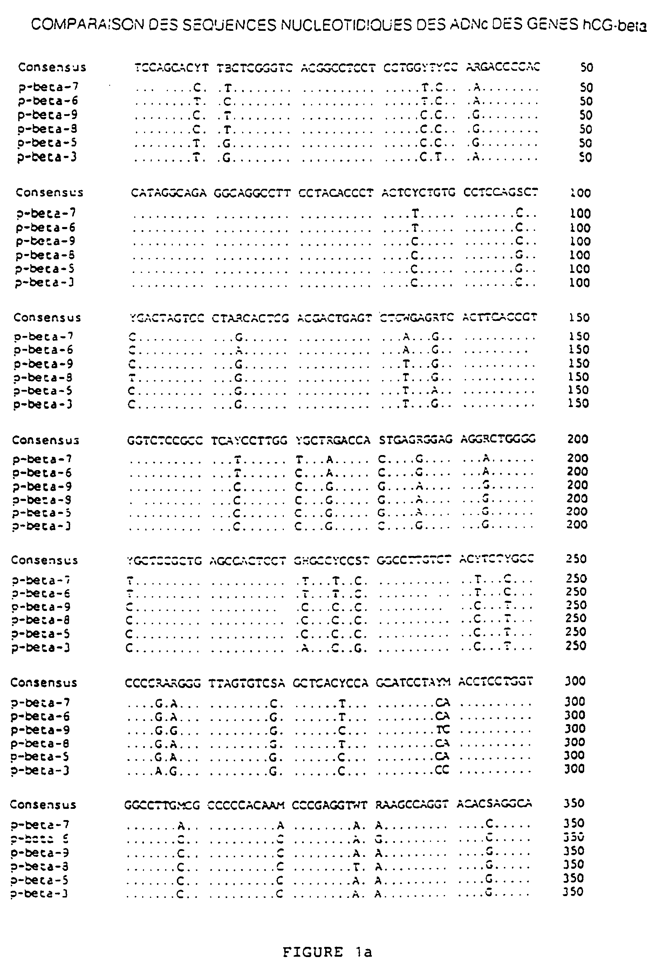

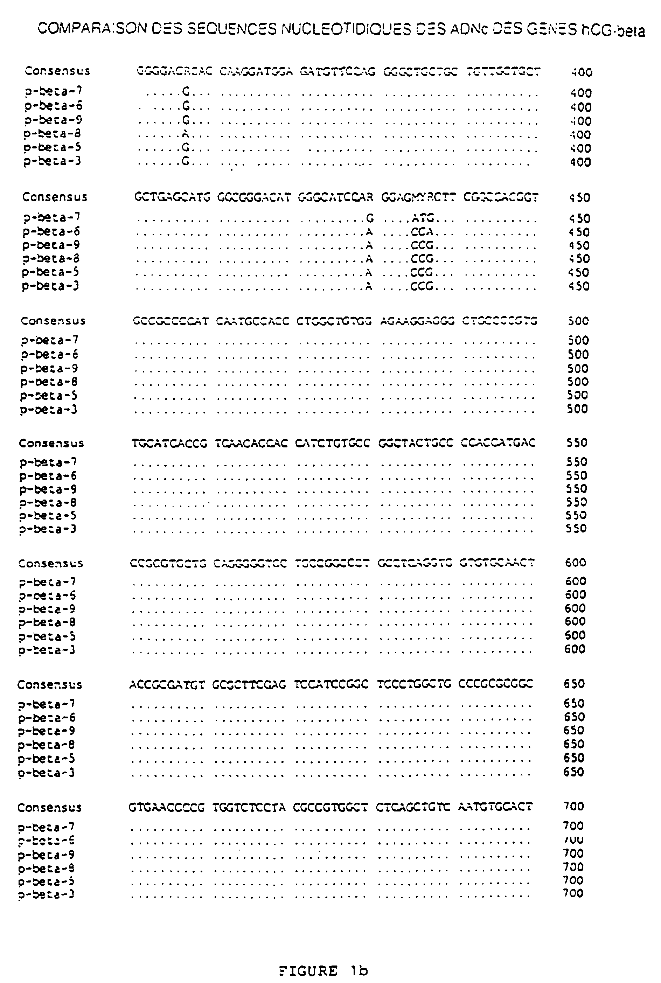

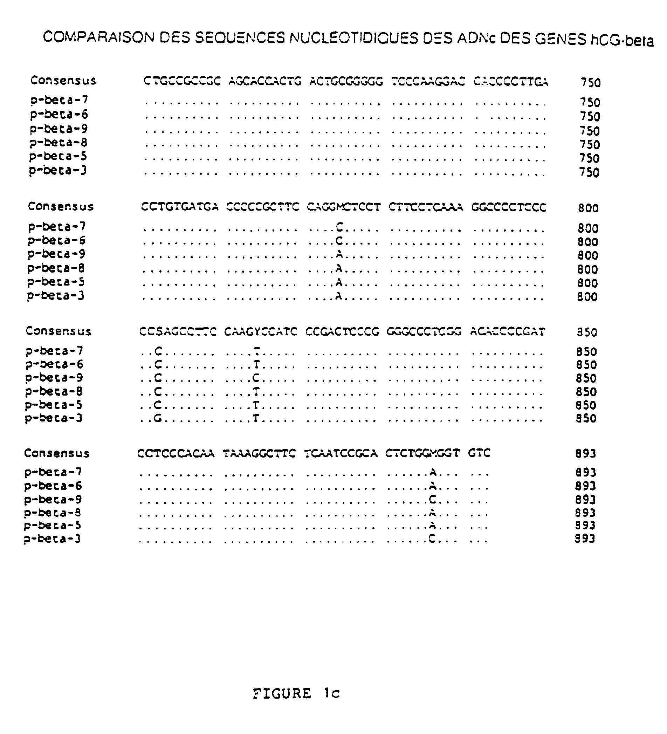

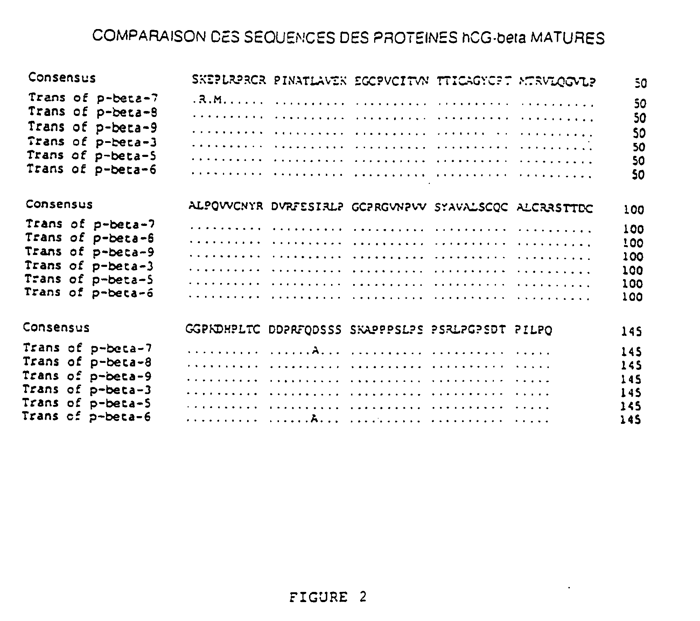

Dans ce qui va suivre, les références des séquences correspondent aux figures 1a, 1b et 1c sur lesquelles sont répétées les séquences nucléotidiques des ADNc des gènes hCGβ, et la figure 2 qui représente les séquences des protéines hCGβ matures.

Les exemples ci-après permettront de mettre en évidence d'autres caractéristiques et avantages du procédé selon la présente invention.

MATERIEL ET METHODES

Extraction des ARN totaux

Les ARN totaux ont été extraits selon la méthode de Chomczynski et Sacchi (20). Les ARN totaux ont été quantifiés par spectrophotométrie UV à 260 nm (1 unité de DO correspondant à 40 µg/ml d'ARN). La qualité des ARN a été appréciée par visualisation, après électrophorèse sur gel d'agarose à 1% et coloration au bromure d'éthidium, des bandes correspondant aux ARN ribosomiques 18S et 28S. Les ARN totaux ont été conservés dans de l'eau DEPC à -80°C.

Choix des amorces oligonucléotidiques

L'analyse des séquences des différents gènes du groupe multigénique hCG/LHβ a permis de sélectionner un couple d'amorces oligonucléotidiques permettant l'amplification sélective et avec la même efficacité des transcrits CGβ3, β5, β6, β7, β8 et β9.

Les séquences nucléotidiques des deux amorces sont les suivantes (les positions sont indiquées par rapport à l'ARNm mature, le site d'initiation de la transcription étant numéroté +1).

Amorce CGI (532-551)

5'-GCTACCTGCCCCACCATGACC-3'

Amorce CGII (878-857)
5'-CGGGATTGAGAAGCCTTTATTGT-3'

Elles permettent l'amplification par RT-PCR à partir d'ARN total d'un fragment de 347 nucléotides.

Pour éliminer l'amplification de l'ADN génomique contaminant les préparations d'ARN, l'amorce CGI a été placée dans une région correspondant à la jonction des exons 2 et 3. Les trois derniers nucléotides de l'amorce CGI correspondent aux trois premières bases de l'exon 3 : ACC. Or, la séquence des trois premières bases de l'intron 2 est GTG pour les gènes CGβ1, β2, β3, β5, β6, β7, β8, β9 et LHβ.

ARN CGβ1, β2, β3, β5, β6, β7, β8 et β9:

Exon 2 Exon3
5'...GGC TAC TGC CCC ACC ATG ACC...3'
Gly Tyr Cys Pro Thr Met Thr

ADN génomique CGβ1, β2, β3, β5, β6, β7, β8, β9 et LHβ :

Exon 2 Intron 2
5'...GGC TAC TGC CCC ACC ATG gtg...3'
Gly TYr Cys Pro Thr Met

L'utilisation de l'amorce CGI permet également d'éliminer l'amplification de l'ARN LHβ éventuellement présent. En effet, les trois dernières bases de l'amorce CGI correspondent au triplet ACC qui code le résidu Thréonine 42 de la protéine CGβ mature. Or, la sous-unité β de la LH possède en position 42 un résidu Méthionine codé par le triplet ATG.

ARN CGβ1 β2, β3, β5, β6, β7, β8 et β9:

Exon 2 Exon 3
5'...GGC TAC TGC CCC ACC ATG ACC...3'
Gly Tyr Cys Pro Thr Met Thr

ARN LHβ:

Exon 2 Exon 3
5'...GGC TAC TGC CCC ACC ATG ATG...3'
Gly Tyr Cys Pro Thr Met Met

<u>Réaction de réverse transcription (RT).</u>

1 μg d'ARN total de chaque échantillon a été transcrit de façon inverse sous un volume de 20 μl en utilisant le kit Gene Ampli RNA PCR (Perkin-Elmer) selon les instructions du fabricant. Le mélange réactionnel de chaque

échantillon comporte 20 unités de réverse transcriptase, 50 unités d'inhibiteur de la RNAse, 2,5 mM d'oligodT, 1 mM de chaque déoxyribonucléotide triphosphate (dA, dT, dC, dG), 10 mM DTT, 10 mM Tris-HCl (pH 8,3), 50 mM KCl et 5 mM MgCl2. Les mélanges réactionnels sont incubés successivement dans un "DNA thermal cycler" 9600 (Perkin-Elmer) 10 minutes à 20°C (hybridation de l'oligodT), 30 minutes à 42°C (réverse transcription) et 5 minutes à 99°C (dénaturation de la réverse transcriptase et de l'inhibiteur de RNAse) puis refroidissement rapide à 4°C.

Réaction d'amplification (PCR)

L'équivalent de 500 ng d'ADNc (10 µl du produit de RT) de chaque échantillon et un témoin négatif vérifiant l'absence de contamination croisée par des produits d'amplification ont été amplifiés sous un volume final de 50 µl renfermant 2,5 unités de Taq polymérase (Perkin-Elmer), 200 mM de chaque déoxyribonucléotide triphosphate (dA, dT, dC, dG), 10 pmoles de chacune des amorces CGI et CGII, 10 mM Tris-HCl (pH 8,3), 50 mM KCl et 1,5 mM MgCl2. L'amplification a été réalisée dans un "DNA thermal cycler 9600" (Perkin-Elmer) sous 35 cycles comprenant chacun 30 secondes à 95'C, 30 secondes à 65°C et 30 secondes à 72°C. La qualité de la PCR a été vérifiée par électrophorèse sur gel d'acrylamide à 8% et révélation des produits d'amplification par transillumination aux UV après coloration au bromure d'éthidium.

Caractérisation de la position 775 des ADNc hCGβ

La comparaison des séquences des ADNc des gènes codant la sous-unité β de l'hCG nous a permis de montrer que les transcrits β6 et β7 possèdent en position 775 un résidu Cytosine alors que les transcrits β3, β5, β8 et β9 possèdent en position 775 un résidu Adénine.

On a donc déterminé une stratégie permettant de distinguer ces deux types de transcrit par analyse de la position 775. Cette stratégie est fondée sur l'analyse des produits d'amplification des transcrits hCGβ par amorçage compétitif d'amorces oligonucléotidiques ou technique COP pour "Competitive Oligonucleotide Priming" (21, 22, 23). Cette méthode a été initiée à la fin des années 1990 et est fondée sur l'utilisation en mélange de deux oligonucléotides dont les caractéristiques sont les suivantes :

- séquence nucléotidique : identique à l'exception de la base 3' qui est soit un résidu Cytosine (oligonucléotide CGII1), soit un résidu Adénine (oligonucléotide CGIV) ;
- marquage en 5' : l'oligonucléotide CGIII est marqué en 5' par le fluorophore TET et l'oligonucléotide CGIV est marqué en 5' par le fluorophore FAM.

CGIII : 5'-TET-ACCCCCGCTTCCAGGC-3'

CGIV : 5'-FAM-ACCCCCGCTTCCAGGA-3'.

L'efficacité d'extension à partir d'une amorce est étroitement dépendante de l'appariement en 3'. Lorsqu'il existe un désappariement en 3' l'efficacité d'extension est très diminuée et d'autant plus que le désappariement est de type A:G, G:A, C:C ou G:G (23).

Il a été montré qu'il est possible d'améliorer la spécificité et par la même la sensibilité de la technique COP en remplaçant la Taq polymérase par le fragment de Stoffel (24). Par ailleurs, un certain nombre de paramètres ont été optimisés : quantité de produits de RT-PCR, quantité d'oligonucléotides, concentration en $Mg^{2+}$ et en dNTPs, nature et nombre de cycles (25).

La réaction COP est réalisée sous un volume de 10 µl à partir de 1 µl de RT-PCR diluée au 1/20 dans de l'eau distillée. Elle renferme 2 unités de Taq polymérase, Stoffel Fragment (Perkin-Elmer), 50 µM de chaque déoxyribonucléotide triphosphate (dA, dT, dC, dG), 0,1 pmole de chacun des deux oligonucléotides CGIII et CGIV, 10 mM Tris-HCl (pH 8,3), 10 mM KCl et 1,5 mM $MgCl_2$. Cette réaction est réalisée dans un "DNA thermal cycler 9600" (Perkin-Elmer) sous 5 cycles comprenant chacun 30 secondes à 95°C et 30 secondes à 65°C. Les produits COP obtenus sont analysés sur un séquenceur automatique d'ADN (Perkin-Elmer, modèle 373A) : 2,5 µl de produit COP sont mélangés à 2 µl de bleu de dépôt et 0,5 µl de marqueur de taille Genescan 2500 ROX (Perkin-Elmer) puis soumis à une électrophorèse d'une durée de 1 heure en tampon TBE 1X (2500V, 40 mA, 30W) en gel dénaturant (acrylamide à 8%, urée 6 M, TBE 1X).

Les résultats sont ensuite analysés par le logiciel Genscan 672 (Perkin-Elmer) qui permet non seulement de différencier les fragments par leur taille et la nature du fluorophore fixé en 5' de l'amorce, mais également de calculer pour chaque pic une surface directement proportionnelle au nombre de molécules présentes dans le produit COP.

Ainsi, la présence d'un fragment de 119 paires de bases marqué en 5' par TET signe la présence de transcrits porteurs en position 775 d'un résidu Cytosine. En revanche, la présence d'un fragment de 119 paires de bases marqué

en 5' par FAM signe la présence de transcrits porteurs en position 775 d'un résidu Adénine.

Cette approche présente deux avantages majeurs :

* Il s'agit potentiellement d'une méthode très sensible puisqu'il est possible d'augmenter le nombre de cycles de la réaction COP et ainsi détecter un faible nombre de cellules exprimant les transcrits porteurs en position 775 d'un résidu Adénine et ce d'autant plus que le désappariement en 3' est de type A:G, c'est-à-dire le désappariement le plus défavorable pour une extension d'amorce.
* Cette méthode est non seulement qualitative mais également quantitative. Le tableau 2 rassemble les résultats d'une expérience réalisée à partir d'un mélange en proportion connue de 2 produits de RT-PCR provenant d'un tissu sain exprimant exclusivement les transcrits 775 Cytosine (échantillon 1, 2 et 3) et un tissu tumoral exprimant majoritairement les transcrits 775 Adénine (échantillons 16, 17, 18). L'expérience pour chaque point a été réalisée en triplicate.

Tableau 2

| Echantillons | Transcrit $GC^{775}C$ (CGIII) | Transcrit $GA^{775}C$ (CGIV) | Indice * |
|---|---|---|---|
| 1 | 20.428 | - | 0 |
| 2 | 22.174 | - | 0 |
| 3 | 25.875 | - | 0 |
| 4 | 15.653 | 2.237 | 12 |
| 5 | 21.960 | 3.414 | 12 |
| 6 | 20.968 | 3.192 | 13 |
| 7 | 16.587 | 5.987 | 26 |
| 8 | 17.438 | 5.923 | 25 |
| 9 | 15.567 | 5.371 | 25 |
| 10 | 13.962 | 10.308 | 42 |
| 11 | 12.117 | 9.335 | 43 |
| 12 | 9.556 | 7.859 | 45 |
| 13 | 8.203 | 13.784 | 62 |
| 14 | 8.076 | 13.335 | 62 |
| 15 | 8.072 | 13.624 | 62 |
| 16 | 503 | 18.779 | 97 |
| 17 | 440 | 15.879 | 97 |
| 18 | 635 | 18.203 | 96 |

* Indice exprimé comme précédemment décrit

Les résultats montrent une excellente reproductibilité et l'existence d'une proportionnalité entre les quantités de transcrit 775A et 775C et l'indice.

On a évalué un certain nombre de cancers et le tableau 3 rassemble les résultats préliminaires concernant les cancers du sein, de la vessie, de la thyroïde, de la prostate et du colon.

Tableau 3

| Indice CG 117 | | | |
|---|---|---|---|
| | | Négatif Indice = 0 | Positif Indice > 0 |
| Sein | Normal : n = 11 | 11(100 %) | 0(0 %) |
| | Tumoral : n = 102 | 58 (57 %) | 44 (43 %) |

Tableau 3 (suite)

| Indice CG 117 | | | |
|---|---|---|---|
| | | Négatif Indice = 0 | Positif Indice > 0 |
| Vessie | Normal : n = 4 | 4 (100 %) | 0(0%) |
| | Tumoral : n = 34 | 13 (38 %) | 21(62 %) |
| Thyroïde | Adénome : n = 15 | 15 (100 %) | 0 (0 %) |
| | Carcinome : n = 12 | 9 (75 %) | 3 (25 %) |
| Prostate | Normal : n = 4 | 4 (100 %) | 0 (0 %) |
| | Tumoral : n = 3 | 2 (66 %) | 1 (33 %) |
| Colon | Normal : n = 1 | 1 (100 %) | 0 (0 %) |
| | Tumoral : n = 2 | 1 (50%) | 1 (50%) |

LISTE DE SEQUENCES

(1) INFORMATIONS GENERALES:

(i) DEPOSANT:

(A) NOM: INSTITUT GUSTAVE-ROUSSY (IGR)
(B) RUE: 39, RUE CAMILLE DESMOULINS
(C) VILLE: VILLEJUIF CEDEX
(E) PAYS: FRANCE
(F) CODE POSTAL: 94805

(ii) TITRE DE L' INVENTION: PROCEDE POUR LA DETECTION DE LA TRANSFORMATION MALIGNE DE CELLULES HUMAINES

(iii) NOMBRE DE SEQUENCES: 4

(iv) FORME DECHIFFRABLE PAR ORDINATEUR:

(A) TYPE DE SUPPORT: Floppy disk
(B) ORDINATEUR: IBM PC compatible
(C) SYSTEME D' EXPLOITATION: PC-DOS/MS-DOS
(D) LOGICIEL: PatentIn Release #1.0, Version #1.30 (OEB)

(vi) DONNEES DE LA DEMANDE ANTERIEURE:

(A) NUMERO DE LA DEMANDE: FR 9602683
(B) DATE DE DEPOT: 04-MAR-1996

(2) INFORMATIONS POUR LA SEQ ID NO: 1:

(i) CARACTERISTIQUES DE LA SEQUENCE:

(A) LONGUEUR: 16 paires de bases
(B) TYPE: nucléotide
(C) NOMBRE DE BRINS: simple
(D) CONFIGURATION: linéaire

(ii) TYPE DE MOLECULE: ADN

(ix) CARACTERISTIQUE:

(A) NOM/CLE: CGIII

(xi) DESCRIPTION DE LA SEQUENCE: SEQ ID NO: 1:

ACCCCCGCTT CCAGGC 16

(2) INFORMATIONS POUR LA SEQ ID NO: 2:

(i) CARACTERISTIQUES DE LA SEQUENCE:

(A) LONGUEUR: 16 paires de bases
(B) TYPE: nucléotide
(C) NOMBRE DE BRINS: simple
(D) CONFIGURATION: linéaire

(ii) TYPE DE MOLECULE: ADN

(ix) CARACTERISTIQUE:

(A) NOM/CLE: CGIV

(xi) DESCRIPTION DE LA SEQUENCE: SEQ ID NO: 2:

ACCCCCGCTT CCAGGA 16

(2) INFORMATIONS POUR LA SEQ ID NO: 3:

(i) CARACTERISTIQUES DE LA SEQUENCE:

(A) LONGUEUR: 893 paires de bases
(B) TYPE: nucléotide
(C) NOMBRE DE BRINS: simple
(D) CONFIGURATION: linéaire

(ii) TYPE DE MOLECULE: ADNc

(xi) DESCRIPTION DE LA SEQUENCE: SEQ ID NO: 3:

```
TCCAGCACYT TBCTCGGGTC ACGGCCTCCT CCTGGYTYCC ARGACCCCAC CATAGGCAGA    60
GGCAGGCCTT CCTACACCCT ACTCYCTGTG CCTCCAGSCT YGACTAGTCC CTARCACTCG   120
ACGACTGAGT CTCWGAGRTC ACTTCACCGT GGTCTCCGCC TCAYCCTTGG YGCTRGACCA   180
STGAGRGGAG AGGRCTGGGG YGCTCCGCTG AGCCACTCCT GHGCCYCCST GGCCTTGTCT   240
ACYTCTYGCC CCCCRARGGG TTAGTGTCSA GCTCACYCCA GCATCCTAYM ACCTCCTGGT   300
GGCCTTGMCG CCCCCACAAM CCCGAGGTWT RAAGCCAGGT ACACSAGGCA GGGGACRCAC   360
CAAGGATGGA GATGTTCCAG GGGCTGCTGC TGTTGCTGCT GCTGAGCATG GGCGGGACAT   420
GGGCATCCAR GGAGMYRCTT CGGCCACGGT GCCGCCCCAT CAATGCCACC CTGGCTGTGG   480
AGAAGGAGGG CTGCCCCGTG TGCATCACCG TCAACACCAC CATCTGTGCC GGCTACTGCC   540
CCACCATGAC CCGCGTGCTG CAGGGGGTCC TGCCGGCCCT GCCTCAGGTG GTGTGCAACT   600
ACCGCGATGT GCGCTTCGAG TCCATCCGGC TCCCTGGCTG CCCGCGCGGC GTGAACCCCG   660
TGGTCTCCTA CGCCGTGGCT CTCAGCTGTC AATGTGCACT CTGCCGCCGC AGCACCACTG   720
ACTGCGGGGG TCCCAAGGAC CACCCCTTGA CCTGTGATGA CCCCCGCTTC CAGGMCTCCT   780
CTTCCTCAAA GGCCCCTCCC CCSAGCCTTC CAAGYCCATC CCGACTCCCG GGGCCCTCGG   840
ACACCCCGAT CCTCCCACAA TAAAGGCTTC TCAATCCGCA CTCTGGMGGT GTC          893
```

(2) INFORMATIONS POUR LA SEQ ID NO: 4:

(i) CARACTERISTIQUES DE LA SEQUENCE:

(A) LONGUEUR: 145 acides aminés
(B) TYPE: acide aminé
(C) NOMBRE DE BRINS: simple
(D) CONFIGURATION: linéaire

(ii) TYPE DE MOLECULE: protéine

(ix) CARACTERISTIQUE:

(A) NOM/CLE: caractéristique particulière
(B) EMPLACEMENT:2
(D) AUTRES INFORMATIONS:/note= "Xaa signifie Lysine ou Arginine"

(ix) CARACTERISTIQUE:

(A) NOM/CLE: caractéristique particulière
(B) EMPLACEMENT:4
(D) AUTRES INFORMATIONS:/note= "Xaa signifie Proline ou Methionine"

(ix) CARACTERISTIQUE:

(A) NOM/CLE: caractéristique particulière
(B) EMPLACEMENT:117
(D) AUTRES INFORMATIONS:/note= "Xaa signifie Alanine ou Acide Aspartique"

(xi) DESCRIPTION DE LA SEQUENCE: SEQ ID NO: 4:

```
Ser Xaa Glu Xaa Leu Arg Pro Arg Cys Arg Pro Ile Asn Ala Thr Leu
1               5                   10                  15

Ala Val Glu Lys Glu Gly Cys Pro Val Cys Ile Thr Val Asn Thr Thr
            20                  25                  30

Ile Cys Ala Gly Tyr Cys Pro Thr Met Thr Arg Val Leu Gln Gly Val
        35                  40                  45

Leu Pro Ala Leu Pro Gln Val Val Cys Asn Tyr Arg Asp Val Arg Phe
    50                  55                  60

Glu Ser Ile Arg Leu Pro Gly Cys Pro Arg Gly Val Asn Pro Val Val
65                  70                  75                  80

Ser Tyr Ala Val Ala Leu Ser Cys Gln Cys Ala Leu Cys Arg Arg Ser
                85                  90                  95

Thr Thr Asp Cys Gly Gly Pro Lys Asp His Pro Leu Thr Cys Asp Asp
            100                 105                 110

Pro Arg Phe Gln Xaa Ser Ser Ser Ser Lys Ala Pro Pro Pro Ser Leu
        115                 120                 125

Pro Ser Pro Ser Arg Leu Pro Gly Pro Ser Asp Thr Pro Ile Leu Pro
    130                 135                 140

Gln
145
```

BIBLIOGRAPHIE

1 - Pierce J.G., Parsons T.F., *Annu. Rev. Biochem.,* 50, 465-495 (1981).
2 - Marcillac I. et al., *Cancer Res.,* 52, 3901-307 (1992).
3 - Alfthan H., Haglund C., Dabek J. & Stenman U.H., *Clin .Chem.,* 38, 1981-1987 (1992).
4 - Marcillac I., Cottu P., Theodore C., Terrier-Lacombe M., Bellet D. & Droz J-P, *Lancet*, 341, 1354-1355 (1993).
5 - Iles R.K., Jenkins B.J., Oliver R.T.D., Blandy J-P & Chard T., *Br. J. Urol*., 64, 241-244 (1989).
6 - Jameson L.J. & Hollenberg A.N., *Endocrinol. Rev*., 14, 203-221 (1993).
7 - Bo M. & Boime I., *J. Biol. Chem.,* 267, 3179-3184 (1992).
8 - Talmadge K., Vamvakopoulos N.C. & Fiddes J.C., *Nature,* 307, 37-40 (1984).
9 - Talmadge K., Boorstein W.R., Vamvakopoulos N.V., Gething M.J. & Fiddes J.C., *Nucleic. Acids Res.,* 12, 8415-8436 (1984).
10- Policastro P., Ovitt C.E., Hoshina M., Fukuoka H., Boothby M.R. & Boime I., *J. Biol. Chem*., 258, 11492-11499 (1983).
11 - Berger P., Kranewitter W., Madersbacher S., Gerth R., Geley S. & Dirnhofer S., *FEBS Lett*., 343, 229-233 (1994).
12 - Madersbacher S., Kratzik C., Gerth R., Dirnhofer S. & Berger P., *Cancer Res*., 54, 5096-5100 (1994).
13 - Oyasu R., Nan L, Smith P. & Kawamata H., *Arch. Pathol. Lab. Med*., 119, 715-717(1994).
14- Jameson L, Lindell C.M. & Habener J.F., *J. Clin. Endocrinol. Metab*., 64, 319-327(1986).
15 - Acevedo H.F., Tong J.Y. & Hartsock R.J., *Cancer,* 76, 1467-1475 (1995).
16 - Cosgrove D.E, Campain J.A. & Cos G.S., *Biochim. Biophys. Acta*, 1007, 44-54 (1989).
17 - Campain J.A., Gutkin D.W. & Cox G.S., *Mol. Endocrinol*., 7, 1331-1346 (1993).
18 - LazarV. et al., *Cancer Res*., 55, 3735-3738 (1995).
19 - Otani T., Otani F., Krych M., Chaplin D.D., Boime I., *J. Biol. Chem*., 263, 7322-7329 (1988).
20- Chomczynski P. & Sacchi N., *Anal. Biochem*., 162, 156-159 (1987).
21 - Gibbs R.A., Nguyen P.N., Caskey C.T., *Nucleic Acids Res*., 17, 2437-2448 (1989).
22 - Chehab F.F. & Kan Y.W., *Proc. Natl. Acad. Sci*. USA, 86, 9178-9182 (1989).

23 - Huang M.M., Arnheim N., Goodman M.F., *Nucleic Acids Res.*, 20, 4567-4573 (1992).
24 - Tada M., Omata M., Kawai S., Saisho H., Ohto M., Saiki R.K., Sninsky J.J., *Cancer Res.*, 53, 2472-2474 (1993).
25 - Rychlik W., *Biotechniques*, 1, 84-89 (1995).

**Revendications**

1. Procédé pour la détection de la transformation maligne de cellules humaines, **caractérisé en ce qu'**on met en évidence dans lesdites cellules une surexpression des produits des gènes β3, β5, β8 et/ou β9 codant pour la sous-unité hCGβ par rapport à leur expression dans les cellules non malignes.

2. Procédé selon la revendication 1, **caractérisé en ce qu'**on met en évidence l'augmentation dans les cellules malignes du rapport entre les produits d'expression des gènes β3, β5, β8 et/ou β9 et les produits d'expression de l'ensemble des gènes β.

3. Procédé selon la revendication 1, **caractérisé en ce qu'**on met en évidence la surexpression de la protéine hCGβ comportant un résidu acide aspartique en position 117.

4. Procédé selon la revendication 3, **caractérisé en ce que** la production de la protéine hCGβ comportant un résidu acide aspartique en position 117 est mise en évidence à l'aide d'un anticorps monoclonal spécifique de l'épitope comportant un acide aspartique en position 117.

5. Procédé selon la revendication 1, **caractérisé en ce qu'**on met en évidence la présence des transcrits des gènes β3, β5, β8 et/ou β9 comportant la séquence codant pour l'acide aspartique en position 774 à 777.

6. Procédé selon la revendication 5, **caractérisé en ce qu'**on met en évidence la présence de transcrits comportant une adénine en position 775.

7. Procédé selon la revendication 6, **caractérisé en ce qu'**on met en évidence la surtranscription dans les cellules malignes d'un ARNm comportant un A en position 775 par rapport à l'ensemble des ARNm des gènes codant pour la sous-unité β.

8. Procédé selon l'une des revendications 5 à 7, **caractérisé en ce qu'**on évalue l'augmentation de l'indice des transcrits comportant un A en position 775 et donc la séquence GAC au codon 117 par rapport à l'ensemble des transcrits de la sous-unité βCG.

9. Procédé selon l'une des revendications 5 à 8, **caractérisé en ce que** pour mettre en évidence la surexpression des transcrits :

   (a) on effectue d'abord une réverse transcription des ARNm totaux,
   (b) on amplifie sélectivement les ADNc des gènes codant la sous-unité β de l'hCG, et
   (c) on met en évidence la présence de produits d'amplification comportant un A en position 775.

10. Procédé selon la revendication 9, **caractérisé en ce qu'**on met en évidence le rapport produit d'amplification comportant un A en position 775 / l'ensemble des produits de l'amplification sélective.

11. Procédé selon l'une des revendications 9 et 10, **caractérisé en ce qu'**on utilise pour la mise en évidence des produits d'amplification comportant un C en position 775 une amorce d'extension comportant un A en position 3' et une autre amorce d'extension comportant un C en position 5', chacune desdites amorces étant identifiables après extension.

12. Procédé selon la revendication 11, **caractérisé en ce que** les deux amorces d'extension sont :

CGIII : 5'-ACCCCCGCTTCCAGGC-3'
CGIV : 5'-ACCCCCGCTTCCAGGA-3'.

## Patentansprüche

1. Verfahren zum Nachweis der malignen Transformation von menschlichen Zellen, **dadurch gekennzeichnet, dass** man in den Zellen eine Überexpression der Produkte der Gene β3, β5, β8 und/oder β9, die für die Untereinheit hCGβ kodieren, bezüglich deren Expression in nicht-malignen Zellen nachweist.

2. Verfahren nach Anspruch 1, **dadurch gekennzeichnet, dass** man die Erhöhung des Verhältnisses zwischen den Expressionsprodukten der Gene β3, β5, β8 und/oder β9 und den Expressionsprodukten der Gesamtheit der β-Gene nachweist.

3. Verfahren nach Anspruch 1, **dadurch gekennzeichnet, dass** man die Überexpression des Proteins hCGβ nachweist, das einen Asparaginsäure-Rest in der Position 117 umfasst.

4. Verfahren nach Anspruch 3, **dadurch gekennzeichnet, dass** man die Produktion des Proteins hCGβ, das einen Asparaginsäure-Rest in der Position 117 umfasst, mit Hilfe eines monoklonalen Antikörpers nachweist, welcher für den Epitop spezifisch ist, der eine Asparaginsäure in der Position 117 umfasst.

5. Verfahren nach Anspruch 1, **dadurch gekennzeichnet, dass** man die Anwesenheit der Transcripte der Gene β3, β5, β8 und/oder β9 nachweist, welche die Sequenz umfassen, die für Asparaginsäure in der Position 774 bis 777 kodiert.

6. Verfahren nach Anspruch 5, **dadurch gekennzeichnet, dass** man die Anwesenheit von Transcripten nachweist, die in der Position 775 ein Adenin umfassen.

7. Verfahren nach Anspruch 6, **dadurch gekennzeichnet, dass** man in malignen Zellen die Übertranscription einer mRNA, welche in der Position 775 ein A umfasst, bezüglich der Gesamtheit der mRNA der Gene nachweist, die für die Untereinheit β kodieren.

8. Verfahren nach einem der Ansprüche 5 bis 7, **dadurch gekennzeichnet, dass** man die Erhöhung des Index der Transcripte, die ein A in der Position 775 und demgemäß die Sequenz GAC am Kodon 117 umfassen, bezüglich der Gesamtheit der Transcripte der Untereinheit βCG bewertet.

9. Verfahren nach einem der Ansprüche 5 bis 8, **dadurch gekennzeichnet, dass** man, um die Überexpression der Transcripte nachzuweisen:

   (a) zuerst eine reverse Transcription der gesamten mRNA bewirkt,
   (b) selektiv die cDNA der Gene amplifiziert, die für die Untereinheit β von hCG kodieren, und
   (c) die Anwesenheit der Amplifikationsprodukte nachweist, die in der Position 775 ein A umfassen.

10. Verfahren nach Anspruch 9, **dadurch gekennzeichnet, dass** man das Verhältnis Amplifikationsprodukt, das in Position 775 ein A umfasst/Gesamtheit der Produkte der selektiven Amplifikation bestimmt,

11. Verfahren nach einem der Ansprüche 9 und 10, **dadurch gekennzeichnet, dass** man für die Bestimmung der Amplifikationsprodukte, die in der Position 775 ein C umfassen, einen Verlängerungs-Primer, der in der Position 3' ein A umfasst, und einen anderen Verlängerungs-Primer verwendet, der in der Position 3' ein C umfasst, wobei jeder der Primer nach der Verlängerung identifizierbar ist.

12. Verfahren nach Anspruch 11, **dadurch gekennzeichnet, dass** die zwei Verlängerungs-Primer sind:

CGIII: 5'-ACCCCCGCTTCCAGGC-3'

CGIV: 5'-ACCCCCGCTTCCAGGA-3'.

## Claims

1. Process for detecting the malignant transformation of human cells, **characterized in that** overexpression of the products of the β3, β5, β8 and/or β9 genes, encoding the hCGβ subunit, as compared with their expression in nonmalignant cells, is demonstrated in the said cells.

2. Process according to Claim 1, **characterized in that** the increase in the ratio between the expression products of the β3, β5, β8 and/or β9 genes and the expression products of the totality of the β genes is demonstrated in the malignant cells.

3. Process according to Claim 1, **characterized in that** overexpression of the hCGβ protein which contains an aspartic acid residue in position 117 is demonstrated.

4. Process according to Claim 3, **characterized in that** production of the hCGβ protein which contains an aspartic acid residue in position 117 is demonstrated with the aid of a monoclonal antibody which is specific for the epitope which contains an aspartic acid in position 117.

5. Process according to Claim 1, **characterized in that** the presence of the transcripts of the β3, β5, β8 and/or β9 genes, which contain the sequence encoding aspartic acid in position 774 to 777, is demonstrated.

6. Process according to Claim 5, **characterized in that** the presence of transcripts which contain an adenine in position 775 is demonstrated.

7. Process according to Claim 6, **characterized in that** overtranscription of an mRNA which contains an A in position 775, as compared with the totality of the mRNAs from the genes encoding the β subunit, is demonstrated in the malignant cells.

8. Process according to one of Claims 5 to 7, **characterized in that** the increase in the index of the transcripts which contain an A in position 775, and therefore the sequence GAC at codon 117, as compared with the totality of the βCG subunit transcripts, is assessed.

9. Process according to one of Claims 5 to 8, **characterized in that**, in order to demonstrate the overexpression of the transcripts:

   (a) the total mRNAs are first of all subjected to reverse transcription,
   (b) the cDNAs of the genes encoding the β subunit of hCG are selectively amplified, and
   (c) the presence of amplification products which contain an A in position 775 is demonstrated.

10. Process according to Claim 9, **characterized in that** the amplification product which contains an A in position 775/the totality of the products of the selective amplification ratio is demonstrated.

11. Process according to either of Claims 9 and 10, **characterized in that** an extension primer which contains an A in the 3' position and another extension primer which contains a C in the 5' position, with each of the said primers being identifiable after the extension, are employed for demonstrating the amplification products which contain a C in position 775.

12. Process according to Claim 11, **characterized in that** the two extension primers are:

CGIII : 5'-ACCCCCGCTTCCAGGC-3'
CGIV : 5'-ACCCCCGCTTCCAGGA-3'.

COMPARAISON DES SEQUENCES NUCLEOTIDIQUES DES ADNc DES GENES hCG-beta

```
Consensus   TCCAGCACYT TBCTCGGGTC ACGGCCTCCT CCTGGYTYCC ARGACCCCAC   50
p-beta-7    ........C. .T........ .......... .....T.C.. .A........   50
p-beta-6    ........T. .C........ .......... .....T.C.. .A........   50
p-beta-9    ........C. .T........ .......... .....C.C.. .G........   50
p-beta-8    ........C. .T........ .......... .....C.C.. .G........   50
p-beta-5    ........T. .G........ .......... .....C.C.. .G........   50
p-beta-3    ........T. .G........ .......... .....C.T.. .A........   50

Consensus   CATAGGCAGA GGCAGGCCTT CCTACACCCT ACTCYCTGTG CCTCCAGSCT  100
p-beta-7    .......... .......... .......... ....T..... .......C..  100
p-beta-6    .......... .......... .......... ....T..... .......C..  100
p-beta-9    .......... .......... .......... ....C..... .......C..  100
p-beta-8    .......... .......... .......... ....C..... .......G..  100
p-beta-5    .......... .......... .......... ....C..... .......G..  100
p-beta-3    .......... .......... .......... ....C..... .......C..  100

Consensus   YGACTAGTCC CTARCACTCG ACGACTGAGT CTCWGAGRTC ACTTCACCGT  150
p-beta-7    C......... ...G...... .......... ...A...G.. ..........  150
p-beta-6    C......... ...A...... .......... ...A...G.. .........   150
p-beta-9    C......... ...G...... .......... ...T...G.. ..........  150
p-beta-8    T......... ...G...... .......... ...T...G.. ..........  150
p-beta-5    C......... ...G...... .......... ...T...A.. ..........  150
p-beta-3    C......... ...G...... .......... ...T...G.. ..........  150

Consensus   GGTCTCCGCC TCAYCCTTGG YGCTRGACCA STGAGRGGAG AGGRCTGGGG  200
p-beta-7    .......... ...T...... T...A..... C....G.... ...A......  200
p-beta-6    .......... ...T...... C...A..... C....G.... ...A......  200
p-beta-9    .......... ...C...... C...G..... G....A.... ...G......  200
p-beta-8    .......... ...C...... C...G..... G....A.... ...G......  200
p-beta-5    .......... ...C...... C...G..... G....A.... ...G......  200
p-beta-3    .......... ...C...... C...G..... C....G.... ...G......  200

Consensus   YGCTCCGCTG AGCCACTCCT GHGCCYCCST GGCCTTGTCT ACYTCTYGCC  250
p-beta-7    T......... .......... .T...T..C. .......... ..T...C...  250
p-beta-6    T......... .......... .T...T..C. .......... ..T...C...  250
p-beta-9    C......... .........  .C...C..C. .........  ..C...T...  250
p-beta-8    C......... .......... .C...C..C. .......... ..C...T...  250
p-beta-5    C......... .......... .C...C..C. .......... ..C...T...  250
p-beta-3    C......... .......... .A...C..G. .......... ..C...T...  250

Consensus   CCCCRARGGG TTAGTGTCSA GCTCACYCCA GCATCCTAYM ACCTCCTGGT  300
p-beta-7    ....G.A... ........C. ......T... ........CA ..........  300
p-beta-6    ....G.A... ........G. ......T... ........CA ..........  300
p-beta-9    ....G.G... ........G. ......C... ........TC ..........  300
p-beta-8    ....G.A... ........G. ......T... ........CA ..........  300
p-beta-5    ....G.A... ........G. ......C... ........CA ..........  300
p-beta-3    ....A.G... ........G. ......C... ........CC ..........  300

Consensus   GGCCTTGMCG CCCCCACAAM CCCGAGGTWT RAAGCCAGGT ACACSAGGCA  350
p-beta-7    .......A.. .........A ........A. A......... ....C.....  350
p-beta-6    .......C.. .........C ........A. G......... ....C.....  350
p-beta-9    .......C.. .........C ........A. A......... ....G.....  350
p-beta-8    .......C.. .........C ........T. A......... ....G.....  350
p-beta-5    .......C.. .........C ........A. A......... ....G.....  350
p-beta-3    .......C.. .........C ........A. A......... ....G.....  350
```

FIGURE 1a

COMPARAISON DES SEQUENCES NUCLEOTIDIQUES DES ADNc DES GENES hCG-beta

```
Consensus    GGGGACRCAC CAAGGATGGA GATGTTCCAG GGGCTGCTGC TGTTGCTGCT   400
p-beta-7     ......G... .......... .......... .......... ..........   400
p-beta-6     ......G... .......... .......... .......... ..........   400
p-beta-9     ......G... .......... .......... .......... ..........   400
p-beta-8     ......A... .......... .......... .......... ..........   400
p-beta-5     ......G... .......... .......... .......... ..........   400
p-beta-3     ......G... .......... .......... .......... ..........   400

Consensus    GCTGAGCATG GGCGGGACAT GGGCATCCAR GGAGMYRCTT CGGCCACGGT   450
p-beta-7     .......... .......... .........G ....ATG... ..........   450
p-beta-6     .......... .......... .........A ....CCA... ..........   450
p-beta-9     .......... .......... .........A ....CCG... ..........   450
p-beta-8     .......... .......... .........A ....CCG... ..........   450
p-beta-5     .......... .......... .........A ....CCG... ..........   450
p-beta-3     .......... .......... .........A ....CCG... ..........   450

Consensus    GCCGCCCCAT CAATGCCACC CTGGCTGTGG AGAAGGAGGG CTGCCCCGTG   500
p-beta-7     .......... .......... .......... .......... ..........   500
p-beta-6     .......... .......... .......... .......... ..........   500
p-beta-9     .......... .......... .......... .......... ..........   500
p-beta-8     .......... .......... .......... .......... ..........   500
p-beta-5     .......... .......... .......... .......... ..........   500
p-beta-3     .......... .......... .......... .......... ..........   500

Consensus    TGCATCACCG TCAACACCAC CATCTGTGCC GGCTACTGCC CCACCATGAC   550
p-beta-7     .......... .......... .......... .......... ..........   550
p-beta-6     .......... .......... .......... .......... ..........   550
p-beta-9     .......... .......... .......... .......... ..........   550
p-beta-8     .......... .......... .......... .......... ..........   550
p-beta-5     .......... .......... .......... .......... ..........   550
p-beta-3     .......... .......... .......... .......... ..........   550

Consensus    CCGCGTGCTG CAGGGGGTCC TGCCGGCCCT GCCTCAGGTG GTGTGCAACT   600
p-beta-7     .......... .......... .......... .......... ..........   600
p-beta-6     .......... .......... .......... .......... ..........   600
p-beta-9     .......... .......... .......... .......... ..........   600
p-beta-8     .......... .......... .......... .......... ..........   600
p-beta-5     .......... .......... .......... .......... ..........   600
p-beta-3     .......... .......... .......... .......... ..........   600

Consensus    ACCGCGATGT GCGCTTCGAG TCCATCCGGC TCCCTGGCTG CCCGCGCGGC   650
p-beta-7     .......... .......... .......... .......... ..........   650
p-beta-6     .......... .......... .......... .......... ..........   650
p-beta-9     .......... .......... .......... .......... ..........   650
p-beta-8     .......... .......... .......... .......... ..........   650
p-beta-5     .......... .......... .......... .......... ..........   650
p-beta-3     .......... .......... .......... .......... ..........   650

Consensus    GTGAACCCCG TGGTCTCCTA CGCCGTGGCT CTCAGCTGTC AATGTGCACT   700
p-beta-7     .......... .......... .......... .......... ..........   700
p-beta-6     .......... .......... .......... .......... ..........   700
p-beta-9     .......... .......... .......... .......... ..........   700
p-beta-8     .......... .......... .......... .......... ..........   700
p-beta-5     .......... .......... .......... .......... ..........   700
p-beta-3     .......... .......... .......... .......... ..........   700
```

FIGURE 1b

COMPARAISON DES SEQUENCES NUCLEOTIDIQUES DES ADNc DES GENES hCG-beta

```
Consensus   CTGCCGCCGC AGCACCACTG ACTGCGGGGG TCCCAAGGAC CACCCCTTGA   750
p-beta-7    .......... .......... .......... .......... ..........   750
p-beta-6    .......... .......... .......... .......... . ........   750
p-beta-9    .......... .......... .......... .......... ..........   750
p-beta-8    .......... .......... .......... .......... ..........   750
p-beta-5    .......... .......... .......... .......... ..........   750
p-beta-3    .......... .......... .......... .......... ..........   750

Consensus   CCTGTGATGA CCCCCGCTTC CAGGMCTCCT CTTCCTCAAA GGCCCCTCCC   800
p-beta-7    .......... .......... ....C..... .......... ..........   800
p-beta-6    .......... .......... ....C..... .......... ..........   800
p-beta-9    .......... .......... ....A..... .......... ..........   800
p-beta-8    .......... .......... ....A..... .......... ..........   800
p-beta-5    .......... .......... ....A..... .......... ..........   800
p-beta-3    .......... .......... ....A..... .......... ..........   800

Consensus   CCSAGCCTTC CAAGYCCATC CCGACTCCCG GGGCCCTCGG ACACCCCGAT   850
p-beta-7    ..C....... ....T..... .......... .......... ..........   850
p-beta-6    ..C....... ....T..... .......... .......... ..........   850
p-beta-9    ..C....... ....C..... .......... .......... ..........   850
p-beta-8    ..C....... ....T..... .......... .......... ..........   850
p-beta-5    ..C....... ....T..... .......... .......... ..........   850
p-beta-3    ..G....... ....T..... .......... .......... ..........   850

Consensus   CCTCCCACAA TAAAGGCTTC TCAATCCGCA CTCTGGMGGT GTC          893
p-beta-7    .......... .......... .......... ......A... ...          893
p-beta-6    .......... .......... .......... ......A... ...          893
p-beta-9    .......... .......... .......... ......C... ...          893
p-beta-8    .......... .......... .......... ......A... ...          893
p-beta-5    .......... .......... .......... ......A... ...          893
p-beta-3    .......... .......... .......... ......C... ...          893
```

FIGURE 1c

COMPARAISON DES SEQUENCES DES PROTEINES hCG-beta MATURES

```
Consensus          SKEPLRPRCR PINATLAVEK EGCPVCITVN TTICAGYCPT MTRVLQGVLP    50
Trans of p-beta-7  .R.M...... .......... .......... .......... ..........    50
Trans of p-beta-8  .......... .......... .......... .......... ..........    50
Trans of p-beta-9  .......... .......... .......... .......... ..........    50
Trans of p-beta-3  .......... .......... .......... .......... ..........    50
Trans of p-beta-5  .......... .......... .......... .......... ..........    50
Trans of p-beta-6  .......... .......... .......... .......... ..........    50

Consensus          ALPQVVCNYR DVRFESIRLP GCPRGVNPVV SYAVALSCQC ALCRRSTTDC   100
Trans of p-beta-7  .......... .......... .......... .......... ..........   100
Trans of p-beta-6  .......... .......... .......... .......... ..........   100
Trans of p-beta-9  .......... .......... .......... .......... ..........   100
Trans of p-beta-3  .......... .......... .......... .......... ..........   100
Trans of p-beta-5  .......... .......... .......... .......... ..........   100
Trans of p-beta-6  .......... .......... .......... .......... ..........   100

Consensus          GGPKDHPLTC DDPRFQDSSS SKAPPPSLPS PSRLPGPSDT PILPQ        145
Trans of p-beta-7  .......... ......A... .......... .......... .....        145
Trans of p-beta-8  .......... .......... .......... .......... .....        145
Trans of p-beta-9  .......... .......... .......... .......... .....        145
Trans of p-beta-3  .......... .......... .......... .......... .....        145
Trans of p-beta-5  .......... .......... .......... .......... .....        145
Trans of p-beta-6  .......... ......A... .......... .......... .....        145
```

FIGURE 2